# EUROPÄISCHE PATENTSCHRIFT

(11) **EP 3 513 867 B1**
(45) Veröffentlichungstag und Bekanntmachung des Hinweises auf die Patenterteilung: **10.04.2024**
(21) Anmeldenummer: 19151207.8
(22) Anmeldetag: 10.01.2019
(51) Int. Cl.: B01D 61/24, B01D 61/28, B01D 63/02

(54) **BLUTBEHANDLUNGSMASCHINE MIT EINEM HOHLFASERFILTERMODUL ZUR HORIZONTALEN ANORDNUNG**
BLOOD TREATMENT MACHINE WITH HOLLOW FIBRE FILTER MODULE FOR HORIZONTAL ARRANGEMENT
MACHINE DE TRAITEMENT DU SANG DOTÉE D'UN MODULE FILTRANT À FIBRES CREUSES DESTINÉ À L'AGENCEMENT HORIZONTAL

(30) Priorität: 11.01.2018 DE 102018100568
(43) Veröffentlichungstag der Anmeldung: 24.07.2019
(73) Patentinhaber: B. Braun Avitum AG, 34212 Melsungen (DE)
(72) Erfinder: RITTER, Kai-Uwe, 34212 Melsungen (DE)
(74) Vertreter: Winter, Brandl - Partnerschaft mbB

(56) Entgegenhaltungen:
- EP-A2- 0 297 970
- EP-B1- 0 297 970
- WO-A1-2017/048224
- DE-A1-102011 107 980
- US-A- 4 179 380
- US-A1- 2017 021 082

## Beschreibung

Die vorliegende Erfindung betrifft eine extrakorporale Blutbehandlungsmaschine, etwa zur Durchführung einer Dialysebehandlung. Gattungsgemäße Blutbehandlungsmaschinen werden eingesetzt, um eine Blutbehandlung /-reinigung durchführen zu können.

### Hintergrund der Erfindung

Der Einsatz von Hohlfaserfiltermodulen zur Blutbehandlung /-reinigung in extrakorporalen Blutbehandlungsmaschinen ist weit verbreiteter Standard. In jüngerer Zeit wird mehr und mehr ein Augenmerk darauf gelegt, die jeweiligen Hohlfaserfiltermodule in kompakter Anordnung mit der Blutbehandlungsmaschine, wie einer Dialysemaschine, zu koppeln, sodass sich die Längen der Leitungen reduzieren.

Für eine kompakte Anordnung ist es zielführend, das Hohlfaserfiltermodul in verschiedenen Betriebslagen flexibel an der Maschine anbringen zu können.

### Stand der Technik

Die Blutbehandlungsmaschinen und Hohlfaserfiltermodule aus dem Stand der Technik sind fast ausschließlich für eine rein vertikale Betriebslage optimiert und somit nicht flexibel in ihrer Position verstellbar / variierbar. Die US 2015/0238676 A1 offenbart eine Blutbehandlungsmaschine zur Durchführung einer Blutbehandlung, mit einer Maschinenfront, an der ein Hohlfaserfiltermodul, in einer horizontalen Lage angeordnet ist, wobei das Hohlfaserfiltermodul ein im Wesentlichen zylindrisches Gehäuse, ein einen Blutzuflussstutzen und einen Blutabflussstutzen aufweisenden Blutraum und ein einen quer zur Längsrichtung des Hohlfaserfiltermoduls verlaufenden Lösungszuflussstutzen und einen quer zur Längsrichtung des Hohlfaserfiltermoduls verlaufenden Lösungsabflussstutzen aufweisenden Lösungsraum, der mit dem Blutraum zumindest abschnittweise semi-permeabel in Verbindung steht, aufweist.

Die EP 0 923 955 B1 offenbart ein Verfahren zur Herstellung eines Dialysators, der für eine vertikale Anordnung angepasst ist. Hierbei liegt das Augenmerk darauf, die Durchlässigkeit der Membran dahingehend zu optimieren, dass eine (Blut-)Reinigung effizient durchführbar ist.

Die DE 10 2011 107 980 A1 offenbart ein horizontal liegend betriebenes Filtermodul, bei welchem eine Höhendifferenz zwischen Lösungszuflussstutzen und Lösungsabflussstutzen vorliegt. Der Lösungszuflussstutzen ist dabei in Längsrichtung des Hohlfaserfiltermoduls angeordnet, der Lösungsabflussstutzen zeigt nach oben und ist quer zur Längsrichtung des Filtermoduls angeordnet.

Weitere Dialysatoren an einer Dialysemaschine sind aus den Dokumenten US 7,776,219 B2 und DE 27 33 280 A1 bekannt.

Weiterer Stand der Technik ist aus den Dokumenten US 4 148 606, US 5 480 565, US 4 179 380, WO 2017/048224 A1 und US 2017/0021082 A1 bekannt.

Versuche der Anmelderin haben im Stand der Technik Nachteile offenbart: Die bisher aus dem Stand der Technik bekannten Hohlfaserfiltermodule sind für den vertikalen Einsatz in Dialysemaschinen vorgesehen und dafür optimiert. Bei einer Verwendung in horizontaler Lage weisen diese zum Teil erhebliche Probleme auf. So können sich in dieser Lage beispielsweise Luftblasen im sog. Blutraum und/oder im Lösungsraum des Hohlfasermoduls ansammeln. Nicht-abtransportierte Luftblasen beeinträchtigen die Reinigungskapazität des Hohlfaserfiltermoduls und sind daher zu vermeiden. Überdies lässt sich eine Entleerung der Lösung (etwa nach einem Primen) nicht ohne zusätzlichen Aufwand durchführen. Folglich sind die aus dem Stand der Technik bekannten Blutbehandlungsmaschinen und Hohlfaserfiltermodule nicht für eine flexible Anordnung geeignet, welche eine hohe Kompaktheit der gesamten Blutbehandlungsmaschine mit sich bringt.

### Kurzbeschreibung der Erfindung

Der vorliegenden Erfindung liegt die Aufgabe zugrunde, die Nachteile aus dem Stand der Technik zu beheben oder zumindest zu mildern, und insbesondere eine solche Blutbehandlungsmaschine zu offenbaren, welche die Vorteile von verschiedenen Anordnungen - nämlich der vertikalen, der horizontalen und einer Mischung aus beiden - miteinander verbindet. Insbesondere zielt die Erfindung also darauf ab, ein Hohlfaserfiltermodul bereit zu stellen, welches zum ersten eine hocheffiziente Blutreinigung, einen Abtransport / ein Entweichen von Luftblasen sowie ein Entleeren von Dialysierflüssigkeit ermöglicht und welches zum zweiten das Aufrüsten einer Blutbehandlungsmaschine mit Schläuchen und dem Hohlfaserfiltermodul ("disposables") vereinfacht und ein hohes Maß an Kompaktheit sowie an Flexibilität ermöglicht.

Die Erfindung widmet sich ebenso der Aufgabenstellung, eine Blutbehandlungsmaschine zu offenbaren, welche eine zuverlässige Blutreinigung bei minimalem Bauraum und einem übersichtlichen und intuitiv verständlichen Maschinenfront-Layout ermöglicht.

Dies wird erfindungsgemäß mittels einer Blutbehandlungsmaschine mit den Merkmalen des Patentanspruchs 1 gelöst. Vorteilhafte Ausführungsformen sind Gegenstand der Unteransprüche.

Aus dieser erfindungsgemäßen Ausgestaltung eines Hohlfaserfiltermoduls und einer Blutbehandlungsmaschine lassen sich etwa folgende weitere Vorteile ableiten:
- die Blutbehandlungsmaschine nimmt aufgrund der optimierten Maschinenfront mit der horizontalen Hohlfaserfiltermodul-Anordnung einen verminderten Bauraum ein und ist flexibel anpassbar;
- das Aufrüsten der Blutbehandlungsmaschine ist aufgrund des klar definierten Platzes für jede Komponente schneller möglich;
- Blut- sowie Lösungsschläuche sind kürzer auszugestalten, was zum ersten (ebenfalls) das Aufrüsten der Blutbehandlungsmaschine vereinfacht und zum zweiten den Temperaturverlust in den jeweiligen Schläuchen minimiert;
- das Entleeren des Hohlfaserfiltermoduls von Lösung (oder auch Blut) ist ohne zusätzliches Verschwenken des Hohlfaserfiltermoduls möglich;
- Luftblasen, die sowohl im Blut- als auch im Lösungsraum auftreten (können) werden (trotz der horizontalen Lage) abgeführt.
- eine effiziente Blutreinigung wird von der horizontalen Lage unterstützt.

Der Gegenstand der Erfindung ist demnach eine extrakorporale Blutbehandlungsmaschine zur Durchführung einer Blutbehandlung, insbesondere einer Dialysebehandlung, mit einer Maschinenfront, an der ein Hohlfaserfiltermodul, insbesondere nach Art eines Dialysators, in einer horizontalen Lage angeordnet ist. Das Hohlfaserfiltermodul weist ein im Wesentlichen zylindrisches Gehäuse, ein einen Blutzuflussstutzen und einen Blutabflussstutzen aufweisenden Blutraum und ein einen quer zur Längsrichtung des Hohlfaserfiltermoduls verlaufenden Lösungszuflussstutzen und einen quer zur Längsrichtung des Hohlfaserfiltermoduls verlaufenden Lösungsabflussstutzen aufweisenden Lösungsraum, der mit dem Blutraum zumindest abschnittweise semi-permeabel in Verbindung steht, um so für die angestrebte Blutbehandlung angepasst zu sein, auf.

Der Begriff des Gehäuses des Hohlfaserfiltermoduls im Rahmen der Erfindung ist breit auszulegen. Es ist etwa mehrteilig aus einem im Wesentlichen zylindrischen Mittelteil und zwei an den jeweiligen Enden angebrachten Endkappen / Dialysatorkappen aufgebaut.

Mit dem Begriff "quer" sind im Rahmen dieser Erfindung sämtliche Richtungen in der Transversalebene des Hohlfaserfiltermoduls umschrieben. Insbesondere umfasst der Begriff "quer" die beiden Richtungsangaben radial und tangential sowie eine Mischform beider Vektoren.

Gemäß der Erfindung liegt in der horizontalen Lage des Hohlfaserfiltermoduls zwischen dem Lösungszuflussstutzen und dem Lösungsabflussstutzen ein Höhenpotential vor, sodass über den einen der beiden Lösungsstutzen (d. h. über eine der Komponenten Lösungszuflussstutzen und Lösungsabflussstutzen) vor oder nach einer Behandlung eine Lösungsentleerung ermöglicht ist und über den anderen der beiden Lösungsstutzen während der Behandlung ein Luftblasenabtransport ermöglicht ist. Bevorzugt werden hierbei die Lösungsentleerung über den Lösungszuflussstutzen und der Luftblasenabtransport über den Lösungsabflussstutzen realisiert.

Das Höhenpotential zwischen zwei Punkten zeichnet sich folglich dadurch aus, dass zwischen dem einen Punkt und dem anderen Punkt eine potentielle Energie vorliegt, die (in der horizontalen oder sogar der teil-horizontalen Lage) ein Abfließen der Lösung ermöglicht. So vereinigt die Erfindung die sich derzeit ausschließenden Effekte eines effizienten Luftblasenabtransports während der Behandlung und einer Dialysatorentleerung nach oder vor der Behandlung.

In anderen Worten lässt sich die Erfindung strukturell derart beschreiben, dass das Hohlfaserfiltermodul der Blutbehandlungsmaschine im Bereich des Lösungszuflussstutzens eine andere (nämlich eine ein Höhenpotential hervorrufende) Geometrie aufweist, als im Bereich des Lösungsabflussstutzens. Folglich ist das Hohlfaserfiltermodul der Blutbehandlungsmaschine an der Transversalebene gespiegelt asymmetrisch.

Der Lösungszuflussstutzen und der Lösungsabflussstutzen sind jeweils zum (zylindrischen) Gehäuse tangential verlaufend angeordnet. Darüber hinaus zeigen der Lösungszuflussstutzen und der Lösungsabflussstutzen in dieselbe Richtung, um unter Vermeidung zusätzlicher Schläuche direkt mit der Maschinenfront der Blutbehandlungsmaschine koppelbar zu sein.

Die Offenbarung betrifft weiterhin ein Hohlfaserfiltermodul mit einem im Wesentlichen zylindrischen Gehäuse, einem einen Blutzuflussstutzen und einen Blutabflussstutzen aufweisenden Blutraum und einem einen quer zur Längsrichtung des Hohlfaserfiltermoduls verlaufenden Lösungszuflussstutzen und einen quer zur Längsrichtung des Hohlfaserfiltermoduls verlaufenden Lösungsabflussstutzen aufweisenden Lösungsraum, der mit dem Blutraum zumindest abschnittweise semi-permeabel in Verbindung steht. Das Hohlfaserfiltermodul ist in einer horizontalen Lage in einer erfindungsgemäßen extrakorporalen Blutbehandlungsmaschine angeordnet / anbringbar.

Die Offenbarung umfasst weiter die Verwendung eines Hohlfaserfiltermoduls zum horizontalen Einsatz in einer Blutbehandlungsmaschine.

In einer vorteilhaften Ausführungsform der Blutbehandlungsmaschine sind der Blutzuflussstutzen und der Blutabflussstutzen des Hohlfaserfiltermoduls jeweils quer, etwa radial oder tangential, zur Längsrichtung des Hohlfaserfiltermoduls verlaufend angeordnet. Dies senkt den benötigten (axialen) Bauraum des Hohlfaserfiltermoduls an der Maschinenfront der Blutbehandlungsmaschine weiter. Ebenso begünstigt jene Ausführungsform die Betriebssicherheit des Hohlfaserfiltermoduls, da einem Abknicken der daran angeschlossenen Schläuche vorgebeugt wird.

In dieser Ausführungsform kann in der horizontalen Lage des Hohlfaserfiltermoduls weiter bevorzugt zwischen dem Blutzuflussstutzen und dem Blutabflussstutzen ebenfalls ein Höhenpotential vorliegen, sodass über den einen der beiden Blutstutzen eine Blut- oder Primingflüssigkeitsentleerung und über den anderen der beiden Blutstutzen (während der Behandlung) ein Luftblasenabtransport ermöglicht ist. Insbesondere der Luftblasenabtransport auf der Blutseite stellt eine erhebliche Verbesserung dar, da Luftblasen von dem semi-permeablen Austausch über die Membran ausgeschlossen sind. Folglich ermöglicht die Erfindung einen Luftblasenabtransport / ein Luftblasenentweichen sowohl auf der Lösungs- als auch auf der Blutseite - trotz einer (zumindest abschnittsweise) horizontal verlaufenden Anordnung.

Wie bereits beschrieben, sind erfindungsgemäß der Lösungszuflussstutzen und der Lösungsabflussstutzen des Hohlfaserfiltermoduls jeweils zum (zylindrischen) Gehäuse tangential verlaufend angeordnet. Dies erzeugt einen drallbehafteten Lösungsmittelstrom, was die Reinigungskapazität verbessern kann. Der radial außen erfolgende tangentiale Zustrom dient weiter dem Ziel der Erfindung, indem er sich synergistisch mit dem Anordnen eines Höhenpotentials kombinieren lässt.

In einer weiteren vorteilhaften Ausführungsform sind der Blutzuflussstutzen und der Blutabflussstutzen (etwa analog zum Lösungszu- und Lösungsabflussstutzen) jeweils zum zylindrischen Gehäuse tangential verlaufend angeordnet. Dies sichert eine homogene Blutverteilung in der Dialysatorkappe und verhindert Bereiche mit niedriger Flussgeschwindigkeit. Weiter wird das Risiko des Abknickens der Blutschläuche drastisch reduziert. Auch auf die Kompaktheit des Hohlfaserfiltermoduls wirkt sich die tangentiale Anordnung aller Anschlüsse vorteilhaft aus.

Eine hierzu alternative oder zusätzlich bevorzugte Ausführungsform zeichnet sich dadurch aus, dass der Blutzuflussstutzen relativ zum Blutabflussstutzen in der Umfangsrichtung des Hohlfaserfiltermoduls, vorzugsweise um 180°, winkelverdreht ist und einer der beiden Blutstutzen (Blutzuflussstutzen oder Blutabflussstutzen) in der horizontalen Lage des Hohlfaserfiltermoduls nach unten zeigt. Somit lassen sich die Vorteile des Lösungsraums (wie Luftentweichung und Flüssigkeitsentleerung) auf den Blutraum übertragen.

Wie bereits beschrieben, zeigen erfindungsgemäß der Lösungszuflussstutzen und der Lösungsabflussstutzen (unter Beibehaltung des Höhenpotentials) in dieselbe Richtung, um unter Vermeidung zusätzlicher Schläuche direkt mit einer Maschinenfront der Blutbehandlungsmaschine koppelbar zu sein. Unter derselben Richtung wird verstanden, dass die beiden Stutzen parallel ausgerichtet sind, wobei der eine oberhalb und der andere unterhalb der entlang der Längsrichtung des Moduls verlaufenden Mittelachse angeordnet sind. So ermöglicht es diese Ausführungsform, die Schläuche, in denen die Lösung von der Blutbehandlungsmaschine zum Hohlfaserfiltermodul und zurück geführt wird, gänzlich entfallen zu lassen. Die Vorteile dieser Ausgestaltung liegen auf der Hand: das Koppeln des Hohlfaserfiltermoduls reduziert sich auf ein simples Aufstecken des Dialysators auf die Maschinenfront, ohne dass hierfür Schläuche notwendig sind. Weiterhin wird der Materialeinsatz vermindert und das Aufrüsten auch zeitlich verbessert. Letztlich ist auch das Unfallrisiko dadurch verringert, dass keine frei herumhängenden Lösungsschläuche mehr zugegen sind.

Fakultativ ist jene Ausführungsform derart ausgestaltet, dass der Blutzuflussstutzen und der Blutabflussstutzen in dieselbe Richtung zeigen, welche der Richtung der Lösungsstutzen vorzugsweise (um 180°) entgegengesetzt ist. Das bedeutet, dass die vier Stutzen allesamt parallel zueinander verlaufen, während die beiden Lösungsstutzen in die eine Richtung zeigen (d.h. in die eine Richtung hin offen sind) und die beiden Blutstutzen in die entgegengesetzte Richtung zeigen. Dies bewirkt, dass die Lösungsanschlüsse zur Maschine zeigen, um mit dieser direkt koppelbar zu sein und dass die Blutanschlüsse auf den Benutzer zeigen, was deren Länge vermindert und das Aufrüsten vereinfacht.

Alternativ können die Blutanschlüsse auch in dieselbe Richtung zeigen wie die Lösungsanschlüsse (also zur Maschinenfront hin). Somit ist der Anschluss gegen äußere Einwirkungen geschützt, was die Sicherheit erhöht.

Bevorzugt ist die Form des zylindrischen Gehäuses des Hohlfaserfiltermoduls derart kompatibel zu der Form der Maschinenfront oder zumindest der Form einer Dialysatorhalterung ausgestaltet, dass die horizontale Lage des Hohlfaserfiltermoduls definiert oder zentriert hervorrufbar ist. Hierunter wird verstanden, dass die Maschinenfront und das Hohlfaserfiltermodul aufeinander angepasst sind, um in einem Poka-Yoke-Prinzip ein falsches Anordnen des Hohlfaserfiltermoduls zu verhindern. Dies erleichtert das horizontale Koppeln des Hohlfaserfiltermoduls mit der Maschinenfront und garantiert das Anliegen des gewünschten erfindungsgemäßen Höhenpotentials. Das Hohlfaserfiltermodul ist demnach nach Art einer Patrize - Matrize Verbindung aufeinander abgestimmt.

Gemäß der Erfindung ist es vorteilhafterweise möglich, die Blutschläuche, die mit dem Blutzuflussstutzen und dem Blutabflussstutzen verbunden sind, integral mit diesem auszugestalten. Demnach ist eine Blutbehandlungsmaschine gemäß der Erfindung nur noch mit einem Einmal-Artikel (disposable) aufzurüsten, der den Dialysator, den Luftabscheider und die Blutschläuche umfasst, während die Dialysierflüssigkeitsschläuche integral von der Maschinenfront ausgestaltet sind.

Die Offenbarung wird nachstehend anhand bevorzugter Ausführungsbeispiele unter Bezugnahme auf die begleitenden Figuren näher erläutert. Die Figuren sind lediglich schematischer Natur und dienen ausschließlich dem Verständnis der Offenbarung. Die gleichen Elemente sind mit denselben Bezugszeichen versehen. Es zeigen:
- Fig. 1a: eine schematische Seitenansicht eines offenbarungsgemäßen Hohlfaserfiltermoduls in einer ersten Ausführungsform;
- Fig. 1b: eine Ansicht entlang der Längsachse des Hohlfaserfiltermodul aus Fig. 1a;
- Fig. 2a: eine Ansicht entlang der Längsachse auf das Hohlfaserfiltermodul in einer zweiten offenbarungsgemäßen Ausführungsform;
- Fig. 2b: eine schematische Seitenansicht des Hohlfaserfiltermoduls aus Fig. 2a;
- Fig. 2c: eine Ansicht entlang der Längsachse des Hohlfaserfiltermoduls aus Fig. 2a;
- Fig. 3a: eine Ansicht entlang der Längsachse des Hohlfaserfiltermoduls in einer dritten offenbarungsgemäßen Ausführungsform;
- Fig. 3b: eine schematische Seitenansicht des Hohlfaserfiltermoduls aus Fig. 3a;
- Fig. 3c: eine Ansicht entlang der Längsachse des Hohlfaserfiltermoduls aus Fig. 3a;
- Fig. 4a: eine Ansicht entlang der Längsachse des Hohlfaserfiltermoduls in einer vierten erfindungsgemäßen Ausführungsform;
- Fig. 4b: eine schematische Seitenansicht des Hohlfaserfiltermoduls aus Fig. 4a;
- Fig. 4c: eine Ansicht entlang der Längsachse des Hohlfaserfiltermoduls aus Fig. 4a;
- Fig. 5a: eine Ansicht entlang der Längsachse des Hohlfaserfiltermoduls in einer fünften erfindungsgemäßen Ausführungsform;
- Fig. 5b: eine schematische Seitenansicht des Hohlfaserfiltermoduls aus Fig. 5a;
- Fig. 5c: eine Ansicht entlang der Längsachse des Hohlfaserfiltermoduls aus Fig. 5a; und
- Fig. 6: eine schematische Ansicht einer Blutbehandlungsmaschine mit horizontal angeordnetem Hohlfaserfiltermodul.

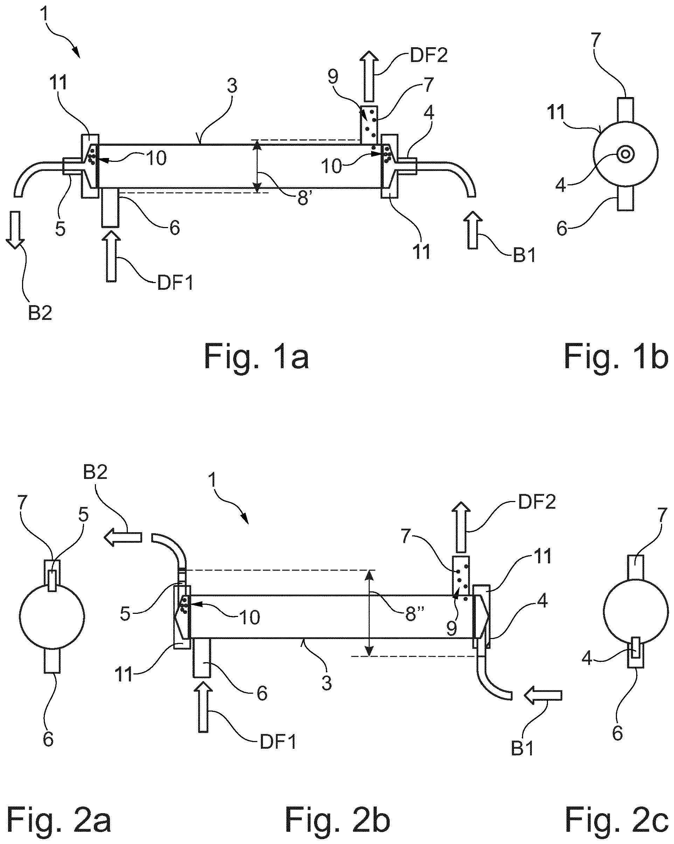

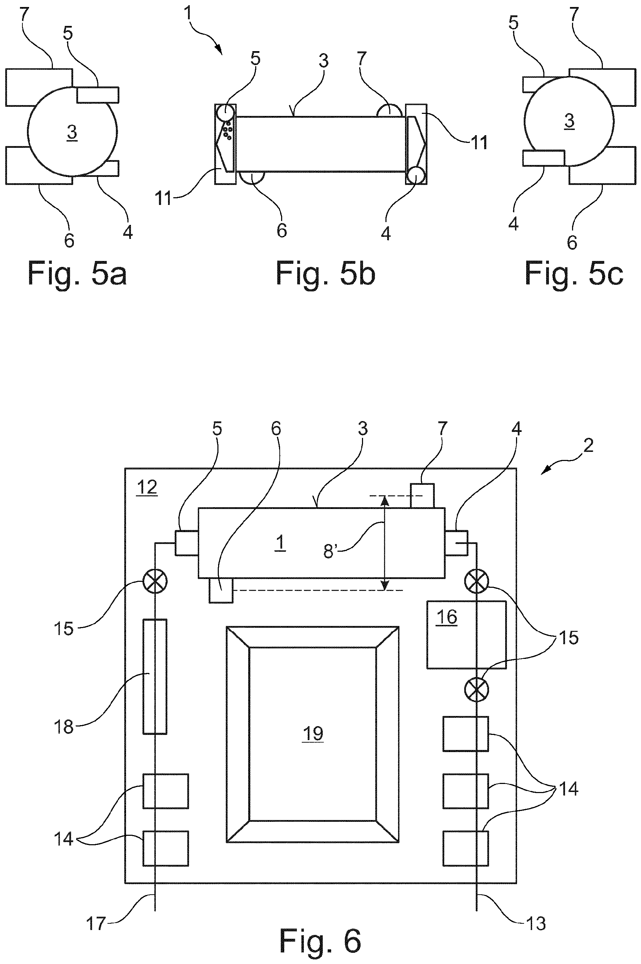

Fig. 1a zeigt ein Hohlfaserfiltermodul 1 nach Art eines Dialysators, der für eine horizontale Anordnung an einer im Zusammenhang mit Fig. 6 gezeigten Blutbehandlungsmaschine 2 angepasst ist. Der Dialysator hat ein im Wesentlichen zylindrisches Gehäuse 3, das einen Blutraum und einen mit diesem zumindest abschnittweise semi-permeabel in Verbindung stehenden Lösungsraum umgibt / ummantelt / umfasst. Über einen Blutzuflussstutzen 4 wird dem Dialysator Blut zugeführt (vgl. Pfeil B1), welches (in gereinigter Form) über einen Blutabflussstutzen 5 wieder von diesem zurück- bzw. abgeführt wird (vgl. Pfeil B2). Ein Lösungszuflussstutzen 6 und ein Lösungsabflussstutzen 7 führen / leiten den Lösungs- bzw. Dialysierflüssigkeitsstrom zum Dialysator hin und von diesem weg (vgl. Pfeile DF1, DF2).

Zwischen dem Lösungszuflussstutzen 6 und dem Lösungsabflussstutzen 7 liegt ein Höhenpotential 8' an. Dieses bewirkt zum ersten, dass in der horizontalen Anordnung des Dialysators zwischen dem Lösungszu- und dem Lösungsabflussstutzen 6, 7 eine Höhendifferenz vorliegt, welche ein Entleeren des Dialysators über den Lösungszuflussstutzen 6 ohne zusätzliche Lageveränderung des Dialysators ermöglicht. Folglich ist für ein Entleeren des Dialysators lediglich das (etwa mittels einer Hansen-Kupplung gekoppelte) Schlauchset vom Dialysator zu lösen und kein zusätzliches Verdrehen des Dialysators oder ähnliches notwendig. Dies vereinfacht die Handhabung und verringert zugleich das Infektionsrisiko, da der Dialysator nur einmal, nämlich zu Beginn, zu berühren ist. Zum zweiten ermöglicht jenes Höhenpotential 8' das Entweichen von Luftblasen 9 aus dem Lösungsraum über den Lösungsabflussstutzen 7 während dem Betrieb. Somit ist die vom Dialysator vorgenommene Blutreinigung hocheffizient und das bei der Verwendung herkömmlicher Dialysatoren bei horizontaler Lage auftretende Problem stehender Luftblasen im Lösungsraum ist gelöst.

Der Lösungszu- und der Lösungsabflussstutzen 6, 7 sind in dem Ausführungsbeispiel aus Fig. 1a zueinander entgegengesetzt ausgerichtet. So sind sie beide radial zum Gehäuse 3 verlaufend angeordnet und um 180° zueinander in Umfangsrichtung winkelversetzt. In der horizontalen Betriebslage ist der Lösungszufluss 6 nach unten zeigend und der Lösungsabfluss 7 nach oben zeigend angeordnet. Dies verstärkt den vorstehend erwähnten Effekt der verbesserten Entleerung bei gleichzeitigem Luftblasenentweichen aus dem Lösungsraum.

Das Gehäuse 3 umfasst neben dem (im Wesentlichen) zylindrischen Abschnitt, an dem die Stutzen 6, 7 angeordnet sind, auch an beiden Stirnseiten jeweils eine Dialysatorkappe 11. Die jeweilige Dialysatorkappe 11 bildet den Blutzu- und den Blutabflussstutzen 4, 5 aus, die vorliegend in der Axialrichtung des Dialysators verlaufend angeordnet sind. Die Dialysatorkappe 11 ist bevorzugt derart ausgestaltet, dass sich die Luftblasen 10 im Blutraum an ihr an- bzw. ablagern können, ohne die Blutreinigung zu beeinträchtigen. Denn im Gegensatz zu den Luftblasen 9 im Lösungsraum sind solche Luftblasen 10 im Blutraum in der ersten Ausführungsform nicht oder nur erschwert abführbar und lagern sich folglich im jeweiligen Endbereich des Gehäuses 3, d. h. im Bereich der Dialysatorkappen 11, an.

Fig. 1b stellt den Dialysator aus Fig. 1a entlang seiner Längsachse dar, woraus die 180°-Anordnung der Stutzen 6, 7 zueinander hervorgeht. Der Blutzuflussstutzen 4, der von der Dialysatorkappe 11 ausgestaltet ist, steht senkrecht auf der gedachten Linie, die den Lösungszuflussstutzen 6 in der vorliegenden Abbildung mit dem Lösungsabflussstutzen 7 verbindet.

Eine zweite Ausführungsform ist in den Figuren 2a bis 2c dargestellt. Fig. 2b stellt den Dialysator jener Ausführungsform in der Seitenansicht dar, während er in Fig. 2a in der Perspektive eines Betrachters auf der einen Seite ("links") und in Fig. 2c in der Perspektive eines Betrachters auf der anderen Seite ("rechts") entlang seiner Längsachse dargestellt ist. Die wesentlichen Komponenten dieser Ausführungsform sind aus der Ausführungsform, welche im Zusammenhang mit Fig. 1a erläutert worden ist, bekannt und seien nun, um Wiederholungen zu vermeiden, nicht nochmals im Detail aufgeführt.

Der Unterschied jener zweiten Ausführungsform zur ersten besteht darin, dass der Blutzuflussstutzen 4 ebenso wie der Blutabflussstutzen 5 radial (und nicht mehr axial) zum Gehäuse 3 verlaufend angeordnet sind. Somit ist neben dem Höhenpotential 8', das im Lösungsraum vorherrscht, auch ein Höhenpotential 8" im Blutraum realisiert.

Dies spart axialen Bauraum ein und vermindert die Abknickgefahr der jeweiligen Schläuche. Außerdem ist nun ein Entweichen / Abtransportieren der Luftblasen 10 im Blutraum ebenso möglich wie ein Entweichen / Abtransportieren der Luftblasen 9 im Lösungsraum.

Wie aus den Figuren 2a und 2c hervorgeht, sind die jeweiligen Blutzu- und Blutabflussstutzen 4, 5 zueinander um 180° in Umfangsrichtung winkelversetzt angeordnet. Die Lösungszu- und der Lösungsabflussstutzen 6, 7 stimmen in ihrer Position in Umfangsrichtung (nicht in Axialrichtung, vgl. Fig. 2b) mit der Position der Blutzu- und Blutabflussstutzen 4, 5 überein. Dem bei Dialysatoren üblichen Gegenstromprinzip Folge leistend, sind in einem Endbereich des Gehäuses 3 der Lösungszuflussstutzen 6 und der Blutabflussstutzen 5 angeordnet, während im anderen, entgegengesetzten Endbereich der Lösungsabflussstutzen 7 und der Blutzuflussstutzen 4 angeordnet sind. Dies gilt für sämtliche hierin offenbarten Ausführungsformen.

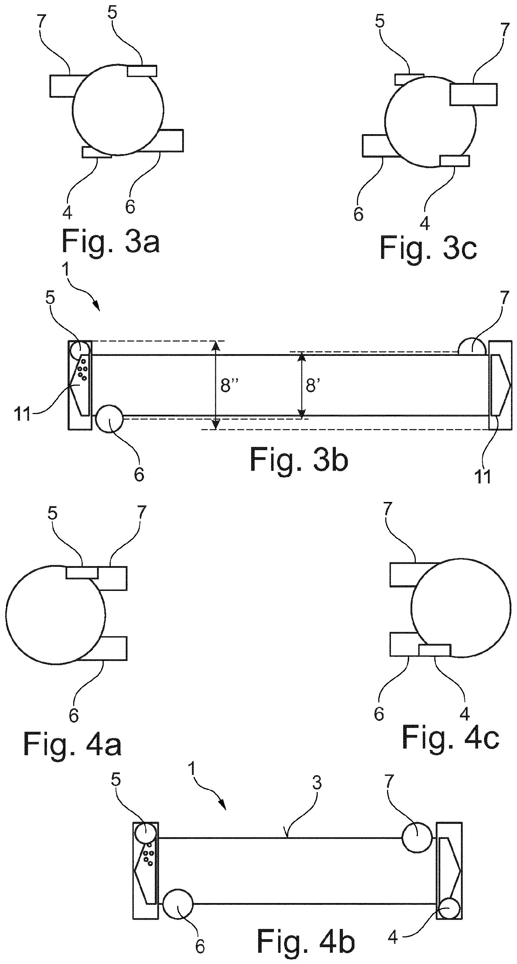

Eine dritte Ausführungsform ist in den Figuren 3a bis 3c gezeigt. Fig. 3b stellt den Dialysator jener Ausführungsform in der Seitenansicht dar, während er in Fig. 3a in der Perspektive eines Betrachters auf der einen Seite ("links") und in Fig. 3c in der Perspektive eines Betrachters auf der anderen Seite ("rechts") entlang seiner Längsachse dargestellt ist. Die wesentlichen Komponenten dieser Ausführungsform sind aus den vorherigen Ausführungsformen bekannt und seien nun, um Wiederholungen zu vermeiden, nicht nochmals im Detail aufgeführt.

Die dritte Ausführungsform unterscheidet sich von den Ausführungsformen der Figuren 1 und 2 darin, dass sämtliche Stutzen 4, 5, 6, 7 zum Gehäuse 3 tangential verlaufend angeordnet sind. Der Blutzuflussstutzen 4 ist folglich in der Ansicht aus Fig. 3b nicht sichtbar. Der Blutaustritt aus dem Blutabflussstutzen 5 tritt in Fig. 3b perspektivisch aus der Zeichenebene heraus, während der Lösungsmitteleintritt in den Lösungszuflussstutzen 6 in die Zeichenebene hinein verläuft.

Jenes tangentiale Ein- und Ausströmen fördert die Verwirbelung der Flüssigkeitsströme und wirkt sich positiv auf die Reinigungsrate des Dialysators aus. Bevorzugt bezieht sich die tangentiale Anordnung auf den (äußeren) Umfang des Dialysators. Wie etwa aus den Figuren 3a und 3c hervorgeht, umfasst der offenbarungsgemäße Gedanke auch solche tangentiale Anordnungen, die nicht ganz außen, sondern etwa bis zum halben Radius tangential angeordnet sind.

In den Figuren 3a und 3c sind sämtliche Stutzen 4, 5, 6, 7 aus der Perspektive des jeweiligen entlang der Längsachse des Dialysators schauenden Betrachters sichtbar. In der vorliegenden Ausführungsform sind die Blutstutzen 4, 5 ebenso wie die Lösungsstutzen 6, 7 jeweils zueinander diagonal gegenüberliegend angeordnet. Folglich ist neben dem Höhenpotential 8', 8" auch eine unterschiedliche Eintritts- und Austrittposition in der Breite erreicht.

So ragen an dem einen Ende des Dialysators die Stutzen 5 und 6 in eine Richtung (aus der Zeichenebene heraus) quer / tangential hervor, während an dem anderen Ende des Dialysators die Stutzen 4 und 7 in die andere Richtung (in die Zeichenebene hinein) quer / tangential hervorragen.

Der Betrag des blutseitigen Höhenpotentials 8" übersteigt den Betrag des lösungsseitigen Höhenpotentials 8', da die Blutstutzen 4, 5 weiter außen anordenbar sind, da die Dialysatorkappen 11 radial weiter herausragen als der Mittelteil des Gehäuses 3. Abweichungen sind möglich und können durch die Position der Lösungsstutzen 6, 7 bzw. der Blutstutzen 4, 5 hervorgerufen werden.

Eine erfindungsgemäße Ausführungsform ist in den Figuren 4a bis 4c dargestellt. Fig. 4b stellt den Dialysator jener Ausführungsform in der Seitenansicht dar, während er in Fig. 4a in der Perspektive eines Betrachters auf der einen Seite ("links") und in Fig. 4c in der Perspektive eines Betrachters auf der anderen Seite ("rechts") dargestellt ist. Die wesentlichen Komponenten dieser Ausführungsform sind aus den vorherigen Ausführungsformen bekannt und seien nun, um Wiederholungen zu vermeiden, nicht nochmals im Detail aufgeführt.

Die Ausführungsform aus Fig. 4 unterscheidet sich von der aus Fig. 3 darin, dass sämtliche tangential verlaufenden Stutzen 4, 5, 6, 7 auf / aus derselben Seite des Dialysators zueinander parallel verlaufend hervorstehen. Somit ist der Dialysator jener Ausführungsform sehr kompakt, wie aus den Figuren 4a und 4c hervorgeht. Der Dialysator ist wahlweise derart mit einer im Zusammenhang mit Fig. 6 dargestellten Maschinenfront 12 koppelbar, dass sämtliche Anschlüsse auf diese hin zeigen (was eine kurze Schlauchlänge bewirkt) oder eben von ihre weg zeigen (was das Aufrüsten der Maschine erleichtert).

Eine weitere erfindungsgemäße Ausführungsform ist in den Figuren 5a bis 5c dargestellt. Fig. 5b stellt den Dialysator jener Ausführungsform in der Seitenansicht dar, während er in Fig. 5a in der Perspektive eines Betrachters auf der einen Seite ("links") und in Fig. 5c in der Perspektive eines Betrachters auf der anderen Seite ("rechts") entlang seiner Längsachse dargestellt ist. Die wesentlichen Komponenten dieser Ausführungsform sind aus den vorherigen Ausführungsformen bekannt und seien nun, um Wiederholungen zu vermeiden, nicht nochmals im Detail aufgeführt.

Das Unterscheidungsmerkmal der Ausführungsform aus Fig. 5 zu jenen aus den vorstehend vorgestellten Ausführungsformen liegt darin, dass die Blutstutzen 4, 5 in die eine Richtung zeigend angeordnet sind, während die Lösungsstutzen 6, 7 in die entgegengesetzte Richtung zeigend angeordnet sind. Vorzugsweise zeigen die Lösungsstutzen 6, 7 zur Maschinenfront 12 (siehe Fig. 6) hin, sodass diese direkt mit der Behandlungsmaschine 2 ohne Zwischenschaltung von Schläuchen koppelbar sind, wie dies vorstehend bereits erläutert worden ist.

In Fig. 6 ist eine Blutbehandlungsmaschine 2 schematisch dargestellt. Die Maschinenfront 12 ist hierbei raumoptimiert ausgestaltet, was maßgeblich aufgrund der horizontalen Anordnung des Hohlfaserfiltermoduls 1 ermöglicht ist. So passiert ein arterieller Schlauch 13, der einem Patienten Blut entnimmt, zunächst Sicherheitseinrichtungen 14 (wie eine Absperrklemme und/oder einen Luft-Detektor), Druckabnehmer 15 sowie eine Pumpe 16 (vorzugsweise nach Art einer Peristaltikpumpe), bevor er mit dem Blutzuflussstutzen 4 verbunden dem Hohlfaserfiltermodul 1 zu reinigendes Blut zuführt.

Nach der Reinigung (im Gegenstromprinzip) tritt das Blut aus dem Blutabflussstutzen 5 heraus in einen venösen Schlauch 17, welcher das Blut (nach weiteren Detektoren sowie einem Luftabscheider 18) gereinigt zum Patienten zurückführt. Ein zentraler Bereich 19 auf der Maschinenfront 12 ist etwa für das Anordnen einer Heparin-Pumpe und für verschiedene Schnittstellen (Anschlüsse und Schalter) freigehalten.

Die horizontale Anordnung des Hohlfaserfiltermoduls 1 ermöglicht zum ersten einen hufeisenförmigen Verlauf der Blutschläuche 13, 17. Somit sind diese möglichst kurz gehalten, was neben den Material- und Platzeinsparungen auch den positiven Effekt eines verminderten Bluttemperaturverlustes in der extrakorporalen Reinigung bewirkt. Weiterhin ermöglicht die horizontale Anordnung, dass der Lösungszuflussstutzen 6 und der Lösungsabflussstutzen 7 unter Realisierung des Höhenpotentials 8' unmittelbar mit der Maschinenfront koppelbar sind, was Lösungs- / Dialysierflüssigkeitsschläuche komplett entfallen lässt.

Abschließend sei noch erwähnt, dass die Blutschläuche (über sämtliche Ausführungsformen hinweg) entweder, etwa über Luer-Anschlüsse, lösbar von dem Dialysator ausgestaltet sind oder aber integral mit den Dialysatorkappen 11 ausgestaltet sind, um die Anzahl an Einweg-Artikeln weiter zu senken.

### Bezugszeichenliste

- 1: Hohlfaserfiltermodul
- 2: Blutbehandlungsmaschine
- 3: Gehäuse
- 4: Blutzuflussstutzen
- 5: Blutabflussstutzen
- 6: Lösungszuflussstutzen
- 7: Lösungsabflussstutzen
- 8': Höhenpotential (lösungsseitig)
- 8": Höhenpotential (blutseitig)
- 9: Luftblasen im Lösungsraum
- 10: Luftblasen im Blutraum
- 11: Dialysatorkappe
- 12: Maschinenfront
- 13: Arterieller Schlauch
- 14: Sicherheitseinrichtungen
- 15: Druckabnehmer
- 16: Pumpe
- 17: Venöser Schlauch
- 18: Luftabscheider
- 19: Zentraler Bereich

- DF1: Lösungszufluss
- DF2: Lösungsabfluss
- B1: Blutzufluss
- B2: Blutabfluss

## Patentansprüche

1. Extrakorporale Blutbehandlungsmaschine (2) zur Durchführung einer Blutbehandlung, mit einer Maschinenfront (12), an der ein Hohlfaserfiltermodul (1), in einer horizontalen Lage angeordnet ist, wobei das Hohlfaserfiltermodul (1)
- ein im Wesentlichen zylindrisches Gehäuse (3),
- einen einen Blutzuflussstutzen (4) und einen Blutabflussstutzen (5) aufweisenden Blutraum und
- einen einen quer zur Längsrichtung des Hohlfaserfiltermoduls (1) verlaufenden Lösungszuflussstutzen (6) und einen quer zur Längsrichtung des Hohlfaserfiltermoduls (1) verlaufenden Lösungsabflussstutzen (7) aufweisenden Lösungsraum, der mit dem Blutraum zumindest abschnittweise semi-permeabel in Verbindung steht, aufweist, wobei in der horizontalen Lage des Hohlfaserfiltermoduls (1) zwischen dem Lösungszuflussstutzen (6) und dem Lösungsabflussstutzen (7) ein Höhenpotential (8') vorliegt, sodass über den einen der beiden Lösungsstutzen (6, 7) eine Lösungsentleerung und über den anderen der beiden Lösungsstutzen (6, 7) ein Luftblasenabtransport ermöglicht ist,
**dadurch gekennzeichnet, dass**
der Lösungszuflussstutzen (6) und der Lösungsabflussstutzen (7) jeweils zum zylindrischen Gehäuse (3) tangential verlaufend angeordnet sind, und
der Lösungszuflussstutzen (6) und der Lösungsabflussstutzen (7) in dieselbe Richtung zeigen, um unter Vermeidung zusätzlicher Schläuche direkt mit der Maschinenfront (12) der Blutbehandlungsmaschine (2) koppelbar zu sein.

2. Extrakorporale Blutbehandlungsmaschine (2) nach Anspruch 1, wobei der Blutzuflussstutzen (4) und der Blutabflussstutzen (5) am Hohlfaserfiltermodul (1) jeweils quer zur Längsrichtung des Hohlfaserfiltermoduls (1) verlaufend angeordnet sind.

3. Extrakorporale Blutbehandlungsmaschine (2) nach Anspruch 2, wobei in der horizontalen Lage des Hohlfaserfiltermoduls (1) zwischen dem Blutzuflussstutzen (4) und dem Blutabflussstutzen (5) ein Höhenpotential (8") vorliegt, sodass über den einen der beiden Blutstutzen (4, 5) eine Blutentleerung und über den anderen der beiden Blutstutzen (4, 5) ein Luftblasenabtransport ermöglicht ist.

4. Extrakorporale Blutbehandlungsmaschine (2) nach einem der vorstehenden Ansprüche, wobei der Blutzuflussstutzen (4) und der Blutabflussstutzen (5) jeweils zum zylindrischen Gehäuse (3) tangential verlaufend angeordnet sind.

5. Extrakorporale Blutbehandlungsmaschine (2) nach einem der vorstehenden Ansprüche, wobei der Blutzuflussstutzen (4) und der Blutabflussstutzen (5) in dieselbe Richtung zeigen, welche der Richtung der Lösungsstutzen (6, 7) vorzugsweise entgegengesetzt ist.

6. Extrakorporale Blutbehandlungsmaschine (2) nach einem der vorstehenden Ansprüche, wobei die Form des zylindrischen Gehäuses (3) derart kompatibel zu der Form der Maschinenfront (12) oder zumindest der Form einer Dialysatorhalterung ausgestaltet ist, dass die horizontale Lage des Hohlfaserfiltermoduls (1) definiert oder zentriert hervorrufbar ist.

## Claims

1. An extracorporeal blood treatment machine (2) for carrying out a blood treatment comprising a machine front (12) on which a hollow fiber filter module (1) is arranged in a horizontal position, wherein the hollow fiber filter module (1) has
- a substantially cylindrical housing (3),
- a blood chamber having a blood inlet nozzle (4) and a blood outlet nozzle (5) and
- a solution chamber having a solution inlet nozzle (6) extending transversely to the longitudinal direction of the hollow fiber filter module (1) and a solution outlet nozzle (7) extending transversely to the longitudinal direction of the hollow fiber filter module (1), the solution chamber being semi-permeably communicated at least in portions with the blood chamber, wherein in the horizontal position of the hollow fiber filter module (1), a height potential (8') is present between the solution inlet nozzle (6) and the solution outlet nozzle (7), so that via the one of the two solution nozzles (6, 7) drainage of solution is enabled and via the other one of the two solution nozzles (6, 7) evacuation of air bubbles is enabled,
**characterized in that**
each of the solution inlet nozzle (6) and the solution outlet nozzle (7) is arranged to extend tangentially relative to the cylindrical housing (3), and
the solution inlet nozzle (6) and the solution outlet nozzle (7) point to the same direction so that they can be coupled directly to the machine front (12) of the blood treatment machine (2) while avoiding additional tubes.

2. The extracorporeal blood treatment machine (2) according to claim 1, wherein each of the blood inlet nozzle (4) and the blood outlet nozzle (5) is arranged at the hollow fiber filter module (1) to extend transversely to the longitudinal direction of the hollow fiber filter module (1).

3. The extracorporeal blood treatment machine (2) according to claim 2, wherein in the horizontal position of the hollow fiber filter module (1), a height potential (8") is present between the blood inlet nozzle (4) and the blood outlet nozzle (5) so that via the one of the two blood nozzles (4, 5) drainage of blood is enabled and via the other one of the two blood nozzles (4, 5) evacuation of air bubbles is enabled.

4. The extracorporeal blood treatment machine (2) according to one of the preceding claims, wherein each of the blood inlet nozzle (4) and the blood outlet nozzle (5) is arranged to extend tangentially relative to the cylindrical housing (3).

5. The extracorporeal blood treatment machine (2) according to one of the preceding claims, wherein the blood inlet nozzle (4) and the blood outlet nozzle (5) point to the same direction which is preferably opposed to the direction of the solution nozzles (6, 7).

6. The extracorporeal blood treatment machine (2) according to one of the preceding claims, wherein the shape of the cylindrical housing (3) is configured to be compatible with the shape of the machine front (12) or at least with the shape of a dialyzer holder such that the horizontal position of the hollow fiber filter module (1) can be produced in a defined or centered form.

## Revendications

1. Machine de traitement extracorporel du sang (2) destinée à la réalisation d'un traitement du sang, avec une partie frontale de machine (12), au niveau de laquelle un module filtrant à fibres creuses (1) est disposé dans une position horizontale, dans laquelle le module filtrant à fibres creuses (1) présente
- un boîtier sensiblement cylindrique (3),
- un réservoir à sang présentant un embout d'apport de sang (4) et un embout de drainage de sang (5) et
- un réservoir à solution présentant un embout d'apport de solution (6) s'étendant transversalement à la direction longitudinale du module filtrant à fibres creuses (1) et un embout de drainage de solution (7) s'étendant transversalement à la direction longitudinale du module filtrant à fibres creuses (1), réservoir à solution qui est en liaison semi-perméable avec le réservoir à sang au moins par sections, dans laquelle, dans la position horizontale du module filtrant à fibres creuses (1), se trouve un potentiel d'élévation (8') entre l'embout d'apport de solution (6) et l'embout de drainage de solution (7) de sorte qu'une élimination de solution soit rendue possible via l'un des deux embouts à solution (6, 7) et une évacuation de bulles d'air soit rendue possible via l'autre des deux embouts à solution (6, 7),
**caractérisé en ce que**
l'embout d'apport de solution (6) et l'embout de drainage de solution (7) sont respectivement disposés en s'étendant tangentiellement par rapport au boîtier cylindrique (3) et l'embout d'apport de solution (6) et l'embout de drainage de solution (7) pointent dans la même direction pour pouvoir être couplés directement à la partie frontale de machine (12) de la machine de traitement du sang (2) en évitant des tuyaux supplémentaires.

2. Machine de traitement extracorporel du sang (2) selon la revendication 1, dans laquelle l'embout d'apport de sang (4) et l'embout de drainage de sang (5) sont disposés au niveau du module filtrant à fibres creuses (1) en s'étendant respectivement transversalement à la direction longitudinale du module filtrant à fibres creuses (1).

3. Machine de traitement extracorporel du sang (2) selon la revendication 2, dans laquelle, dans la position horizontale du module filtrant à fibres creuses (1) se trouve un potentiel d'élévation (8") entre l'embout d'apport de sang (4) et l'embout de drainage de sang (5) de sorte qu'une élimination de sang soit rendue possible via l'un des deux embouts à sang (4, 5) et une évacuation de bulles d'air soit rendue possible via l'autre des deux embouts à sang (4, 5).

4. Machine de traitement extracorporel du sang (2) selon l'une quelconque des revendications précédentes, dans laquelle l'embout d'apport de sang (4) et l'embout de drainage de sang (5) sont respectivement disposés en s'étendant tangentiellement par rapport au boîtier cylindrique (3).

5. Machine de traitement extracorporel du sang (2) selon l'une quelconque des revendications précédentes, dans laquelle l'embout d'apport de sang (4) et l'embout de drainage de sang (5) pointent dans la même direction qui est de préférence opposée à la direction des embouts de solution (6, 7).

6. Machine de traitement extracorporel du sang (2) selon l'une quelconque des revendications précédentes, dans laquelle la forme du boîtier cylindrique (3) est compatible avec la forme de la partie frontale de machine (12) ou au moins la forme d'un support d'appareil de dialyse de sorte que la position horizontale du module filtrant à fibres creuses (1) soit actionnable de manière définie ou centrée.
